(19) Europäisches Patentamt
European Patent Office
Office européen des brevets

(11) EP 4 726 725 A1

(12) **EUROPEAN PATENT APPLICATION**
published in accordance with Art. 153(4) EPC

(43) Date of publication:
**15.04.2026 Bulletin 2026/16**

(21) Application number: **24819226.2**

(22) Date of filing: **29.05.2024**

(51) International Patent Classification (IPC):
**G16C 60/00** (2019.01)    **G16C 20/70** (2019.01)

(52) Cooperative Patent Classification (CPC):
**G16C 20/70; G16C 60/00**

(86) International application number:
**PCT/JP2024/019633**

(87) International publication number:
**WO 2024/252995 (12.12.2024 Gazette 2024/50)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **06.06.2023 JP 2023093364**

(71) Applicant: **Dai Nippon Printing Co., Ltd.
Tokyo 162-8001 (JP)**

(72) Inventor: **MURAYAMA Yusuke
Tokyo 162-8001 (JP)**

(74) Representative: **Müller-Boré & Partner
Patentanwälte PartG mbB
Friedenheimer Brücke 21
80639 München (DE)**

(54) **PHYSICAL PROPERTY PREDICTION DEVICE, PHYSICAL PROPERTY PREDICTION METHOD, AND PHYSICAL PROPERTY PREDICTION PROGRAM**

(57)    A physical property prediction apparatus includes an actual measurement data acquisition unit that acquires actual measurement data including a first manufacturing condition for a sample and an actually measured value obtained by actually measuring a physical property of the sample manufactured on the basis of the first manufacturing condition, a virtual data acquisition unit that acquires virtual data including a second manufacturing condition for the sample and a virtual value obtained by virtualizing a physical property of the sample that corresponds to the second manufacturing condition, and a model generation unit that, on the basis of the actual measurement data acquired by the actual measurement data acquisition unit and the virtual data acquired by the virtual data acquisition unit, generates such a prediction model of the physical property that has a first uncertainty of the physical property with respect to the actual measurement data and a second uncertainty of the physical property with respect to the virtual data. The second uncertainty of the sample is greater than the first uncertainty of the sample.

FIG. 1

EP 4 726 725 A1

**Description**

Technical Field

The present disclosure relates to a physical property prediction apparatus, a physical property prediction method, and a physical property prediction program.

Background Art

In recent years, attention has been paid to a technical filed called "materials informatics", which is intended to improve the efficiency of material development by making use of information science technology such as machine learning. In materials informatics, it is necessary to find out appropriate combinations of a large number of parameters pertaining to compounding ratios of raw materials and process conditions in order to manufacture a material having a desired physical property. In a case where there are a large number of parameters, there are numerous combinations of parameters. Numerous combinations of parameters make it difficult to comprehensively prepare samples of a material. In a case where it is difficult to comprehensively prepare samples of a material, a method is adopted in which the physical properties of a given number of prepared samples of a material are measured and a prediction model of physical properties is constructed on the basis of actual measurement data obtained by measurement. Using a prediction model makes it possible to search for optimum parameters even if there are only a limited number of samples of a material.

To construct a prediction model, a supervised machine learning technique may be used. With a supervised machine learning technique, it is difficult to construct a high-accuracy prediction model in a case where there are only an insufficient number of actual measurement data or a case where a relationship between parameters and physical properties is complicated. To construct a high-accuracy model even in a case where there are only an insufficient number of actual measurement data or a case where a relationship between parameters and properties is complicated, a technique of sequential design of experiments such as Bayesian optimization is used. Bayesian optimization involves the use of Bayesian inference to construct a prediction model. Bayesian inference is a method based on the idea of Bayesian probability in which a matter to be inferred is statistically inferred from an observed phenomenon. Bayesian probability is probability interpreted as a rational expected value representing a state of knowledge or as quantification of a personal creed. In Bayesian inference, a predicted value of a physical property and a uncertainty of the predicted value are calculated. The uncertainty thus calculated makes it possible to calculate, for example, the probability with which the physical property assumes a particular value and an expected value of the physical property. Bayesian inference makes it possible to find optimum parameters on the basis of limited actual measurement data. That is, Bayesian inference makes it possible to build up an experimental design for the next sample preparation of a material on the basis of limited actual measurement data. A prediction model is updated by the next sample preparation of a material, measurement of a physical property of a prepared sample of the material, and addition of a measured result to actual measurement data. Bayesian optimization involves the repetition of a series of cycles composed of the aforementioned sample preparation, measurement of a physical property, and updating of a prediction model. The repetition of the series of cycles makes it possible to minimize the number of times that sample preparation is conducted until a desired physical property value is attained.

However, in a case where there are only few or no actual measurement data acquired, it is difficult to construct an initial prediction model in Bayesian optimization. When it is difficult to construct an initial prediction model, it is difficult to build up a sequential experimental design. Specifically, the number of actual measurement data required to construct a prediction model normally ranges approximately from 5 to 10. A larger number of parameters may lead to a further increase in the number of actual measurement data required to construct a prediction model. However, in a case where it costs a lot of money and takes a lot of time to prepare samples of a material and measure physical properties or a case where there are only a limited budget and time for development, the number of times that sample preparation can be conducted is limited to a small number. When the number of sample preparations is limited to a small number, there are only an insufficient number of actual measurement data, with the result that it becomes difficult to construct an initial prediction model and apply Bayesian optimization.

WO 2021/200281 A1 discloses a technology with which to make a sequential experimental design by applying machine learning to actual measurement data. WO 2020/188971 A1 discloses a technology with which to learn physical property values in advance by finding physical property values corresponding to parameters through simulation such as quantum-chemistry calculation.

However, in a case where a given number of actual measurement data are not prepared in the initial stage, it is difficult to carry out the technology disclosed in WO 2021/200281 A1. In particular, in a case where the number of sample preparations and the number of measurements of a physical property are limited to small numbers, it is difficult to obtain as many actual measurement data as required to create an initial prediction model.

Further, the technology disclosed in WO 2020/188971 A1 can only be carried out in some fields with established simulation techniques. That is, in many fields with difficulty applying simulation, it is difficult to carry out the technology disclosed in WO 2020/188971 A1.

Disclosure of Invention

The present disclosure was made in view of the foregoing points and has as an object to provide a physical property prediction apparatus, a physical property prediction method, and a physical property prediction program that make it possible to appropriately and efficiently develop a material having a designed physical property.

A physical property prediction apparatus according to the present disclosure includes an actual measurement data acquisition unit that acquires actual measurement data including a first manufacturing condition for a sample and an actually measured value obtained by actually measuring a physical property of the sample manufactured on the basis of the first manufacturing condition, a virtual data acquisition unit that acquires virtual data including a second manufacturing condition for the sample and a virtual value obtained by virtualizing a physical property of the sample that corresponds to the second manufacturing condition, and a model generation unit that, on the basis of the actual measurement data acquired by the actual measurement data acquisition unit and the virtual data acquired by the virtual data acquisition unit, generates such a prediction model of the physical property that has a first uncertainty of the physical property with respect to the actual measurement data and a second uncertainty of the physical property with respect to the virtual data. The second uncertainty of the sample is greater than the first uncertainty of the sample.

In the physical property prediction apparatus according to the present disclosure, the virtual data acquisition unit may acquire, as the virtual value, a physical property value of the sample that is predicted in a case where a value of at least one of a plurality of parameters included in the second manufacturing condition is extremely small or large.

In the physical property prediction apparatus according to the present disclosure, the virtual data acquisition unit may acquire, as the virtual value, a physical property value of a substance that is similar in raw material or process to the sample.

In the physical property prediction apparatus according to the present disclosure, the first uncertainty of the physical property may be 0.

The physical property prediction apparatus according to the present disclosure may further include an input screen generation unit that generates an input screen through which to input the actual measurement data and the virtual data. The actual measurement data acquisition unit may acquire the actual measurement data inputted to the input screen generated by the input screen generation unit. The virtual data acquisition unit may acquire the virtual data inputted to the input screen generated by the input screen generation unit.

The physical property prediction apparatus according to the present disclosure may further include a communication unit that sends to a user's terminal device the input screen generated by the input screen generation unit and that receives from the terminal device the actual measurement data and the virtual data inputted to the input screen. The actual measurement data acquisition unit may acquire the actual measurement data received by the communication unit. The virtual data acquisition unit may acquire the virtual data received by the communication unit.

The physical property prediction apparatus according to the present disclosure may further include a virtual data imputation unit that imputes the virtual data. The virtual data acquisition unit may acquire the virtual data imputed by the virtual data imputation unit.

In the physical property prediction apparatus according to the present disclosure, the virtual data imputation unit may impute the second manufacturing condition of the virtual data by, when one or more parameters, included in the plurality of parameters included in the second manufacturing condition, that have extremely small or large values and the virtual value predicted on the basis of the one or more parameters are inputted to the input screen, calculating a value of one of the plurality of parameters other than the one or more parameters.

The physical property prediction apparatus according to the present disclosure may further include an uncertainty setting unit that sets the first uncertainty of the physical property and the second uncertainty of the physical property. The model generation unit may generate the prediction model on the basis of the first uncertainty of the physical property and the second uncertainty of the physical property as set by the uncertainty setting unit.

The physical property prediction apparatus according to the present disclosure may further include a setting screen generation unit that generates a setting screen through which to set the first uncertainty of the physical property and the second uncertainty of the physical property. The uncertainty setting unit may set the first uncertainty of the physical property and the second uncertainty of the physical property in accordance with an input operation performed on the setting screen generated by the setting screen generation unit.

In the physical property prediction apparatus according to the present disclosure, the model generation unit may generate the prediction model using Bayesian inference. The first uncertainty may be a first variance. The second uncertainty may be a second variance that is greater than the first variance.

In the physical property prediction apparatus according to the present disclosure, the model generation unit may generate the prediction model according to the following formulas:

[Math. 1]

$$\theta_i = \sum_{j=1}^{d} \alpha_j \phi_j(x_i) + \varepsilon_i$$

[Math. 2]

$$p\left(\{\alpha_j\}_{j=1}^{d} \middle| \{x_i\}_{i=1}^{n}, \{\theta_i\}_{i=1}^{n}\right) = \frac{p\left(\{\theta_i\}_{i=1}^{n} \middle| \{x_i\}_{i=1}^{n}, \{\alpha_j\}_{j=1}^{d}\right) p\left(\{\alpha_j\}_{j=1}^{d}\right)}{\int p\left(\{\theta_i\}_{i=1}^{n} \middle| \{x_i\}_{i=1}^{n}, \{\alpha_j\}_{j=1}^{d}\right) p\left(\{\alpha_j\}_{j=1}^{d}\right) d\alpha_1 \cdots d\alpha_d}$$

$$= \frac{\left(\prod_{i=1}^{n} p\left(\theta_i \middle| x_i, \{\alpha_j\}_{j=1}^{d}\right)\right) \cdot p\left(\{\alpha_j\}_{j=1}^{d}\right)}{\int \left(\prod_{i=1}^{n} p\left(\theta_i \middle| x_i, \{\alpha_j\}_{j=1}^{d}\right)\right) \cdot p\left(\{\alpha_j\}_{j=1}^{d}\right) d\alpha_1 \cdots d\alpha_d}$$

where $\theta_\ell$ is a physical property in the ith datum of the actual measurement data and the virtual data, $x_i$ is a manufacturing condition in the ith datum of the actual measurement data and the virtual data, $\phi_j(\cdot)$ is a basis function, $\varepsilon_i$ is an error in the physical property in the ith datum of the actual measurement data and the virtual data, $\alpha_j$ is a parameter of the prediction model,

$$p\left(\{\alpha_j\}_{j=1}^{d}\right)$$

is a prior distribution,

$$p\left(\{\alpha_j\}_{j=1}^{d} \middle| \{x_i\}_{i=1}^{n}, \{\theta_i\}_{i=1}^{n}\right)$$

is a posterior distribution,

$$p\left(\theta_i \middle| x_i, \{\alpha_j\}_{j=1}^{d}\right)$$

is a likelihood corresponding to the ith datum and is a value equivalent to $p\left(\theta_i \middle| x_i, \{\alpha_j\}_{j=1}^{d}\right) = N(\theta_i | \sum_{j=1}^{d} \alpha_j \phi_j(x_i), \sigma_i^2)$, $\sigma_i^2$ is an error variance of the physical property in the ith datum of the actual measurement data and the virtual data, and $\int p\left(\{y_i\}_{i=1}^{n} \middle| \{x_i\}_{i=1}^{n}, \{\alpha_j\}_{j=1}^{d}\right) p\left(\{\alpha_j\}_{j=1}^{d}\right) d\alpha_1 \cdots d\alpha_d$

is a marginal likelihood.

The physical property prediction apparatus according to the present disclosure may further include a manufacturing condition calculation unit that calculates a third manufacturing condition for the sample on the basis of the prediction model generated by the model generation unit.

In the physical property prediction apparatus according to the present disclosure, the third manufacturing condition may include at least one of a manufacturing condition where the uncertainties of the physical property in the prediction model reach their maximums and a manufacturing condition where the uncertainties of the physical property in the prediction model reach their minimums.

In the physical property prediction apparatus according to the present disclosure, the actual measurement data acquisition unit may further acquire second actual measurement data including the third manufacturing condition calculated by the manufacturing condition calculation unit and a second actually measured value obtained by actually measuring a physical property of the sample manufactured under the third manufacturing condition. The model generation unit may update the prediction model on the basis of the second actual measurement data acquired by the actual measurement data acquisition unit.

In the physical property prediction apparatus according to the present disclosure, the model generation unit may not update the prediction model in a case where the second actually measured value is a set optimum value.

A physical property prediction method according to the present disclosure includes the steps of acquiring actual measurement data including a first manufacturing condition for a sample and an actually measured value obtained by actually measuring a physical property of the sample manufactured under the first manufacturing condition, acquiring virtual data including a second manufacturing condition for the sample and a virtual value obtained by virtualizing a physical property of the sample that corresponds to the second manufacturing condition, and on the basis of the actual measurement data thus acquired and the virtual data thus acquired, generating such a prediction model of the physical property that has a first uncertainty of the physical property with respect to the actual measurement data and a second uncertainty of the physical property with respect to the virtual data, the second uncertainty of the sample being greater than the first uncertainty of the sample.

A physical property prediction program according to the present disclosure causes a computer to execute procedures comprising the steps of acquiring actual measurement data including a first manufacturing condition for a sample and an actually measured value obtained by actually measuring a physical property of the sample manufactured under the first manufacturing condition, acquiring virtual data including a second manufacturing condition for the sample and a virtual value obtained by virtualizing a physical property of the sample that corresponds to the second manufacturing condition, and on the basis of the actual measurement data thus acquired and the virtual data thus acquired, generating such a prediction model of the physical property that has a first uncertainty of the physical property with respect to the actual measurement data and a second uncertainty of the physical property with respect to the virtual data, the second uncertainty of the sample being greater than the first uncertainty of the sample.

The present disclosure makes it possible to appropriately and efficiently develop a material having a designed physical property.

Brief Description of Drawings

[Fig. 1] Fig. 1 is a schematic view showing a physical property prediction apparatus according to a first embodiment.

[Fig. 2] Fig. 2 is a block diagram showing the physical property prediction apparatus according to the first embodiment.

[Fig. 3] Fig. 3 is a flow chart showing an example of operation of the physical property prediction apparatus according to the first embodiment.

[Fig. 4] Fig. 4 is a diagram showing an example of an input screen in the example of operation of the physical property prediction apparatus according to the first embodiment.

[Fig. 5] Fig. 5 is a diagram showing an example of a prediction model in the example of operation of the physical property prediction apparatus according to the first embodiment.

[Fig. 6] Fig. 6 is a diagram showing another example of a prediction model in the example of operation of the physical property prediction apparatus according to the first embodiment.

[Fig. 7] Fig. 7 is a diagram showing an example of calculation of a third manufacturing condition based on a prediction model in the example of operation of the physical property prediction apparatus according to the first embodiment.

[Fig. 8] Fig. 8 is a block diagram showing a physical property prediction apparatus according to a second embodiment.

[Fig. 9] Fig. 9 is a flow chart showing an example of operation of the physical property prediction apparatus according to the second embodiment.

[Fig. 10] Fig. 10 is a diagram showing an example of an input screen in the example of operation of the physical property prediction apparatus according to the second embodiment.

[Fig. 11] Fig. 11 is a block diagram showing a physical property prediction apparatus according to a third embodiment.

[Fig. 12] Fig. 12 is a flow chart showing an example of operation of the physical property prediction apparatus according to the third embodiment.

[Fig. 13] Fig. 13 is a diagram showing an example of an input screen in the example of operation of the physical property prediction apparatus according to the third embodiment.

[Fig. 14] Fig. 14 is a block diagram showing a physical property prediction apparatus according to a fourth embodiment.

Description of Embodiments

The following describes embodiments of the present disclosure with reference to the drawings. In the drawings that are referred to in the embodiments, identical components or components having similar functions are given identical reference signs or similar reference signs, and a repeated description of such components may be omitted.

[First Embodiment]

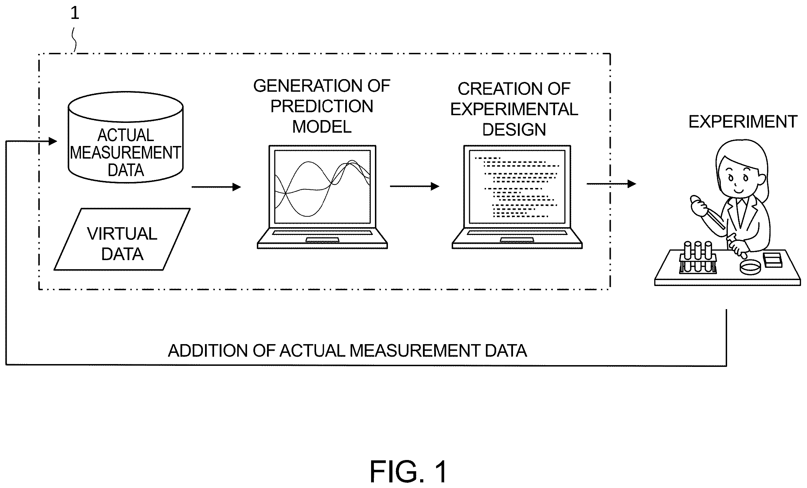

First, a physical property prediction apparatus according to a first embodiment is described with reference to Figs. 1 to 7. Fig. 1 is a conceptual diagram showing a physical property prediction apparatus 1 according to the first embodiment.

As shown in Fig. 1, the physical property prediction apparatus 1 according to the first embodiment generates a prediction model for a sample on the basis of actual measurement data and prediction data. The actual measurement data are data including a first manufacturing condition for the sample and an actually measured value. The sample is, for example, a material manufactured from a plurality of raw materials. Examples of the first manufacturing condition include compounding ratios of the raw materials of the material and process conditions such as temperature. The actually measured value is a value obtained by actually measuring a physical property of a sample actually manufactured under the first manufacturing condition. Examples of the physical property include electric conductivity and a dielectric constant. The prediction data are data including a second manufacturing condition for the sample and a virtual value. The second manufacturing condition is a manufacturing condition that is different from the first manufacturing condition. The second manufacturing condition is not a manufacturing condition where the sample was actually manufactured but a virtualized manufacturing condition. The virtual value is a value obtained by virtualizing a physical property of the sample that corresponds to the second manufacturing condition. The prediction model is information that indicates a physical property of a sample as predicted on the basis of a manufacturing condition for the sample. In other words, the prediction model is information that indicates a relationship of correspondence between a manufacturing condition for a sample and a physical property of the sample.

Further, the physical property prediction apparatus 1 creates a new experimental design for a sample on the basis of the prediction mode thus generated. In accordance with the new experimental design thus created, a new sample is prepared. On the new sample thus prepared, an experiment for actually measuring a physical property value is conducted. Actual measurement data obtained by the experiment is added to the physical property prediction apparatus 1. The physical property prediction apparatus 1 updates the prediction model on the basis of actual measurement data updated by the addition and virtual data.

As mentioned earlier, the physical property prediction apparatus 1 optimizes the experimental design by repeating the generation of a prediction model based on actual measurement data and virtual data, the creation of an experimental design, and the addition of actual measurement data.

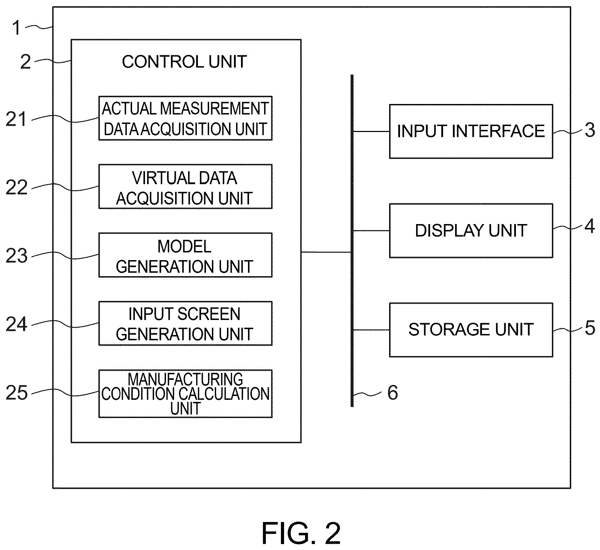

Fig. 2 is a block diagram showing the physical property prediction apparatus 1 according to the first embodiment. More specifically, as shown in Fig. 2, the physical property prediction apparatus 1 includes a control unit 2, an input interface 3, a display unit 4, and a storage unit 5. The control unit 2, the input interface 3, the display unit 4, and the storage unit 5 are communicably connected to one another via a bus 6.

The input interface 3 accepts a variety of instructions and information input operations from a user. The input interface 3 converts into an electric signal an input operation accepted from the user. The input interface 3 outputs to the control unit 2 the input operation converted into the electric signal. Possible examples of the input interface 3 include a mouse, a keyboard, a touchpad, a touch panel, a trackball, a switch button, and a microphone.

The display unit 4 displays an image corresponding to image data sent from the control unit 2. Possible examples of the display unit 4 include a liquid crystal monitor, a CRT (cathode-ray tube) monitor, and a touch panel. The display unit 4 may include a speaker.

The storage unit 5 is a non-transient storage device in which to store various types of information. Possible examples of the storage unit 5 include an HDD (hard disk driver), an optical disc, a magnetic disc, an magneto-optical disc, a CD (compact disc), a DVD (digital versatile disc), and a semiconductor memory. The storage unit 5 has stored therein a physical property prediction program that the physical property prediction apparatus 1 executes and various types of data that are used for executing the physical property prediction program.

The control unit 2 controls, in accordance with an input operation performed by the user using the input interface 3, how the physical property prediction apparatus 1 operates. The control unit 2 includes an actual measurement data acquisition unit 21, a virtual data acquisition unit 22, a model generation unit 23, an input screen generation unit 24, and a manufacturing condition calculation unit 25.

Functions corresponding to the component units 21 to 25 of the control unit 2 are stored in the form of a computer-executable physical property prediction program in the storage unit 5. The control unit 2 is, for example, a processor. By reading out the physical property prediction program from the storage unit 5 and executing the physical property prediction program, the processor constituting the control unit 2 executes the functions of the component units 21 to 25 that correspond to the physical property prediction program. It is possible to incorporate the physical property prediction program directly into a circuit of the processor instead of storing the physical property prediction program in the storage unit 5. In incorporating the physical property prediction program directly, the processor executes the functions corresponding to the component units 21 to 25 by executing the physical property prediction program incorporated into the circuit. The physical property prediction program may be a program that the physical property prediction apparatus 1 downloaded from a server through a network. Alternatively, the physical property prediction program may be a program loaded from a portable storage medium into the physical property prediction apparatus 1. The control unit 2 may be constituted by a single processor. The control unit 2 may be constituted by a combination of multiple processors. Possible examples of the processor include a CPU (central processing unit), a GPU (graphics processing unit), an ASIC (application specific integrated circuit), and a programmable logic device. The programmable logic device may be an SPLD (simple programmable logic device), a CPLD (complex programmable logic device), or an FPGA(field programmable gate array).

The actual measurement data acquisition unit 21 acquires actual measurement data including a first manufacturing condition for a sample and an actually measured value obtained by actually measuring a physical property of the sample manufactured under the first manufacturing condition.

The virtual data acquisition unit 22 acquires virtual data including a second manufacturing condition for the sample and a virtual value obtained by virtualizing a physical property of the sample that corresponds to the second manufacturing condition.

The virtual data acquisition unit 22 may acquire, as the virtual value, a physical property value of the sample that is predicted in a case where a value of one or more of a plurality of parameters included in the second manufacturing condition is extremely small or large.

A case where the value of a parameter is extremely small is a case where the value of the parameter is the value of a lower limit of a predetermined search area of the parameter or a value falling below the lower limit. A case where the value of a parameter is extremely large is a case where the value of the parameter is the value of an upper limit of a predetermined search area of the parameter or a value exceeding the upper limit.

Instead of acquiring a physical property value that is predicted in a case where the value of one or more parameters is extremely small or large, the virtual data acquisition unit 22 may acquire, as the virtual value, a physical property value of a substance that is similar in raw material or process to the sample.

The model generation unit 23 generates a prediction model of the physical property on the basis of the actual measurement data acquired by the actual measurement data acquisition unit 21 and the virtual data acquired by the virtual data acquisition unit 22. The prediction model may be a probabilistic prediction model that indicates a predicted physical property with a probability. The model generation unit 23 generates such a prediction model that has a first uncertainty of the physical property with respect to the actual measurement data and a second uncertainty of the physical property with respect to the virtual data. The second uncertainty of the sample is greater than the first uncertainty of the sample. The first uncertainty of the sample is, for example, 0.

The input screen generation unit 24 generates an input screen through which to input the actual measurement data and the virtual data. The input screen generation unit 24 displays on the display unit 4 the input screen thus generated. The actual measurement data acquisition unit 21 acquires the actual measurement data inputted to the input screen generated by the input screen generation unit 24. More specifically, the actual measurement data acquisition unit 21 acquires the actual measurement data inputted through the input interface 3 to the input screen displayed on the display unit 4. The virtual data acquisition unit 22 acquires the virtual data inputted to the input screen generated by the input screen generation unit 24. More specifically, the virtual data acquisition unit 22 acquires the virtual data inputted through the input interface 3 to the input screen displayed on the display unit 4.

The manufacturing condition calculation unit 25 calculates a third manufacturing condition for the sample on the basis of the prediction model generated by the model generation unit 23.

The third manufacturing condition may include at least one of a manufacturing condition where the uncertainties of the physical property in the prediction model reach their maximums and a manufacturing condition where the uncertainties of the physical property in the prediction model reach their minimums.

The actual measurement data acquisition unit 21 further acquires second actual measurement data including the third manufacturing condition calculated by the manufacturing condition calculation unit 25 and a second actually measured value obtained by actually measuring a physical property of the sample manufactured under the third manufacturing condition.

The model generation unit 23 updates the prediction model on the basis of the second actual measurement data acquired by the actual measurement data acquisition unit 21. The model generation unit 23 does not update the prediction model in a case where the second actually measured value is a set optimum value.

The model generation unit 23 may generate the prediction model using Bayesian inference. The first uncertainty may be a first variance, and the second uncertainty may be a second variance that is greater than the first variance.

In generating the prediction model using Bayesian inference, the model generation unit 23 may generate the prediction model according to the following formulas:

[Math. 3]

$$\theta_i = \sum_{j=1}^{d} \alpha_j \phi_j(x_i) + \varepsilon_i \tag{1}$$

[Math. 4]

$$p\left(\{\alpha_j\}_{j=1}^{d} \middle| \{x_i\}_{i=1}^{n}, \{\theta_i\}_{i=1}^{n}\right) = \frac{p\left(\{\theta_i\}_{i=1}^{n} \middle| \{x_i\}_{i=1}^{n}, \{\alpha_j\}_{j=1}^{d}\right) p\left(\{\alpha_j\}_{j=1}^{d}\right)}{\int p\left(\{\theta_i\}_{i=1}^{n} \middle| \{x_i\}_{i=1}^{n}, \{\alpha_j\}_{j=1}^{d}\right) p\left(\{\alpha_j\}_{j=1}^{d}\right) d\alpha_1 \cdots d\alpha_d}$$

$$= \frac{\left(\prod_{i=1}^{n} p\left(\theta_i \middle| x_i, \{\alpha_j\}_{j=1}^{d}\right)\right) \cdot p\left(\{\alpha_j\}_{j=1}^{d}\right)}{\int \left(\prod_{i=1}^{n} p\left(\theta_i \middle| x_i, \{\alpha_j\}_{j=1}^{d}\right)\right) \cdot p\left(\{\alpha_j\}_{j=1}^{d}\right) d\alpha_1 \cdots d\alpha_d} \tag{2}$$

In mathematical formula (1) and mathematical formula(2), $\theta_\ell$ is a physical property in the ith datum of the actual measurement data and the virtual data, $x_i$ is a manufacturing condition in the ith datum of the actual measurement data and the virtual data, $\phi_j(\cdot)$ is a basis function, $\varepsilon_i$ is an error in the physical property in the ith datum of the actual measurement data and the virtual data, $\alpha_j$ is a parameter of the prediction model,

$$p\left(\{\alpha_j\}_{j=1}^{d}\right)$$

is a prior distribution,

$$p\left(\{\alpha_j\}_{j=1}^{d} \middle| \{x_i\}_{i=1}^{n}, \{\theta_i\}_{i=1}^{n}\right)$$

is a posterior distribution,

$$p\left(\theta_i \middle| x_i, \{\alpha_j\}_{j=1}^{d}\right)$$

is a likelihood corresponding to the ith datum and is a value equivalent to $p\left(\theta_i \middle| x_i, \{\alpha_j\}_{j=1}^{d}\right) = N(\theta_i | \sum_{j=1}^{d} \alpha_j \phi_j(x_i), \sigma_i^2)$, $\sigma_i^2$ is an error variance of the physical property in the ith datum of the actual measurement data and the virtual data, and $\int p\left(\{y_i\}_{i=1}^{n} \middle| \{x_i\}_{i=1}^{n}, \{\alpha_j\}_{j=1}^{d}\right) p\left(\{\alpha_j\}_{j=1}^{d}\right) d\alpha_1 \cdots d\alpha_d$ is a marginal likelihood.

In generating the prediction model according to mathematical formulas (1) and (2), the model generation unit 23 sets to a low value the variance $\sigma^2$ of a likelihood corresponding to the actual measurement data and sets to a high value the variance $\sigma^2$ of a likelihood corresponding to the virtual data. The model generation unit 23 may set to 0 the variance $\sigma^2$ of the likelihood corresponding to the actual measurement data.

The model generation unit 23 may set the prior distribution

$$p\left(\left\{\alpha_j\right\}_{j=1}^{d}\right)$$

so as to have a high probability density in a case where the value of a physical property $\theta$ varies smoothly.

[Example of Operation]

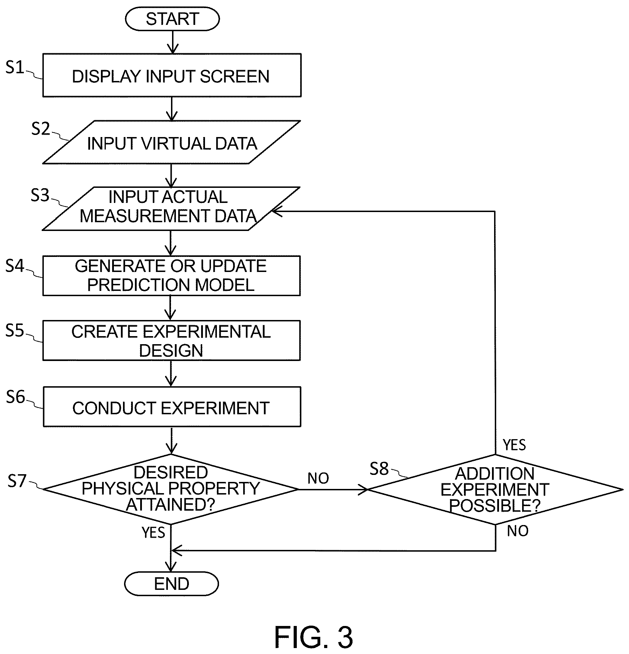

Next, an example of operation of the physical property prediction apparatus 1 thus configured is described. Fig. 3 is a flow chart showing an example of operation of the physical property prediction apparatus 1 according to the first embodiment. It should be noted first that Fig. 3 is based on the premise that a user creates virtual data. The user may create the virtual data by making a prediction based on experience. For example, in a case where each of the plurality of parameters included in the second manufacturing condition assumes an extremely small or large value, the physical property value of the sample may be experientially predicted. In a case where the physical property value of the sample is experientially predicted, the user creates the virtual data by using as the second manufacturing condition the plurality of parameters that assumes an extremely small or large value and using the predicted physical property value of the sample as the virtual value. Alternatively, the user may create the virtual data by using a known physical property value of a substance that is similar in raw material or process to the sample. In a case where a known physical property value of a substance is used, the user creates the virtual data by using a process of the substance as the second manufacturing condition and using the known physical property value of the substance as the virtual value.

Further, the user creates actual measurement data. Specifically, the user creates an actually measured value by measuring a physical property of the sample manufactured under the first manufacturing condition. Then, the user creates the actual measurement data by associating the first manufacturing condition with the actually measured value. In creating the actual measurement data, the user may set approximately one to five combinations of values of a plurality of parameters constituting the first manufacturing condition. Then, the user may create the actual measurement data by actually measuring a physical property of a sample prepared in accordance with a combination of values of the parameters thus set. In setting a combination of values of the plurality of parameters, the user may randomly set the values of the plurality of parameters of the first manufacturing condition within a predetermined range. Alternatively, the user may set the values of the plurality of parameters of the first manufacturing condition by using a method, such as Latin square sampling, in which values of parameters are uniformly selected within a predetermined range.

After the virtual data and the actual measurement data have been created, the input screen generation unit 24 generates an input screen in accordance with an input operation performed by the user using the input interface 3. Then, the input screen generation unit 24 displays on the display unit 4 the input screen thus generated (step S1).

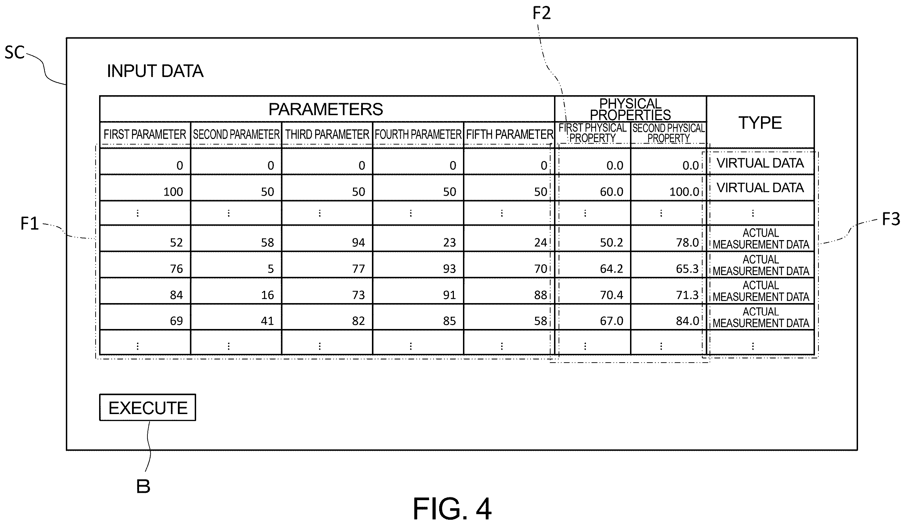

Fig. 4 is a diagram showing an example of an input screen SC in the example of operation of the physical property prediction apparatus 1 according to the first embodiment. In the example shown in Fig. 4, the input screen SC has an input field F1 into which to input first to fifth parameters serving as examples of the first manufacturing condition. Further, the input screen SC has an input field F2 into which to input a first physical property and a second physical property serving as examples of the actually measured values and the virtual value. Further, the input screen SC has an input field F3 into which to input a type of data. i.e. either the virtual data or the actual measurement data. By pressing an execute button B after inputting data into the input fields F1 to F3, the data inputted into the input fields F1 to F3 are confirmed.

After the input screen has been displayed on the display unit 4, as shown in Fig. 3, the user uses the input interface 3 to perform an input operation to input the virtual data to the input screen displayed on the display unit 4 (step S2).

After inputting the virtual data, the user uses the input interface 3 to perform an input operation to input the actual measurement data to the input screen displayed on the display unit 4 (step S3). Step S2 may follow step S3 instead of preceding step S3.

After the virtual data and the actual measurement data have been inputted, the model generation unit 23 generates a prediction model on the basis of the virtual data and the actual measurement data thus inputted (step S4). In the example shown in Fig. 3, the model generation unit 23 generates the prediction model using Bayesian inference according to mathematical formulas (1) and (2) described above. In generating the prediction model, the model generation unit 23 sets to 0 the variance $\sigma^2$ of the likelihood corresponding to the actual measurement data. Further, the model generation unit 23 sets, to a value greater than 0, the variance $\sigma^2$ of the likelihood corresponding to the virtual data. Further, the model generation unit 23 may set the prior distribution p(A) so as to have a high probability density in a case where the value of a physical property θ varies smoothly.

The model generation unit 23 may display on the display unit 4 the prediction model thus generated.

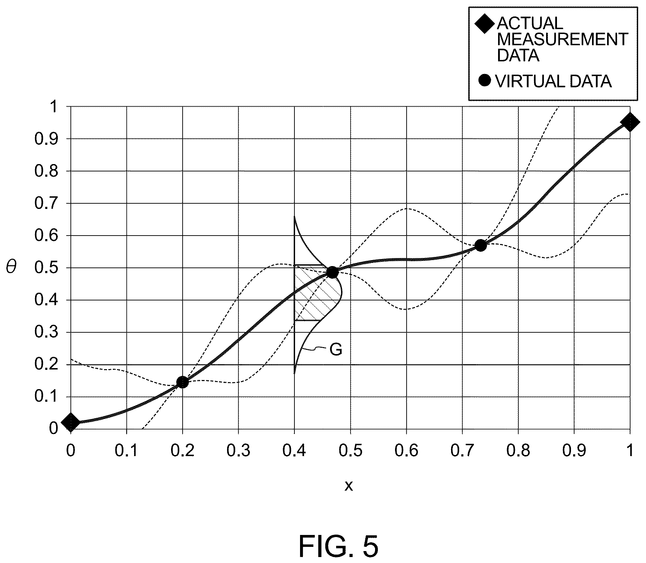

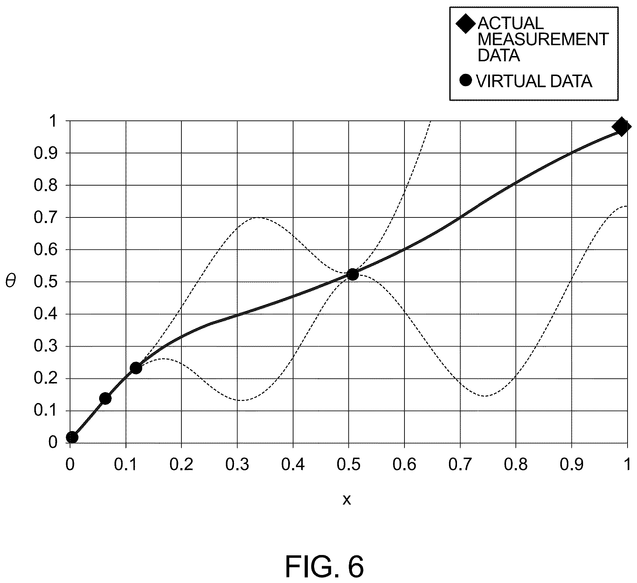

Fig. 5 is a diagram showing an example of a prediction model in the example of operation of the physical property prediction apparatus 1 according to the first embodiment. Fig. 6 is a diagram showing another example of a prediction model in the example of operation of the physical property prediction apparatus 1 according to the first embodiment. The model generation unit 23 generates, for example, the prediction models shown in Figs. 5 and 6. In each of Figs. 5 and 6, the horizontal axis represents a parameter x serving as an example of the first manufacturing condition. In each of Figs. 5 and 6, the vertical axis represents a physical property value θ. In each of Figs. 5 and 6, the solid line in the graph represents an average value of the physical property θ that corresponds to the parameter x. The actual measurement data and the virtual data are located on the solid line in the graph that represents the average value. In each of Figs. 5 and 6, the two dashed lines in the graph represent uncertainties of the physical property θ. Specifically, each of Figs. 5 and 6, the two dashed lines in the graph represent the upper and lower limits, respectively, of a 50% confidence interval in a normal distribution serving as a probability distribution of the physical property θ. As one example, in Fig. 5, the normal distribution G at x - 0.4 is shown in association with the solid line in the graph that represents the average value and the dashed lines in the graph that represent the uncertainties. A shaded area in the normal distribution G corresponds to the 50% confidence interval at x = 0.4.

As can be seen from Figs. 5 and 6, the uncertainties are 0 at points near the actual measurement data. The uncertainties are 0 because the variance $\sigma^2$ of the likelihood corresponding to the actual measurement data has been set to 0 by the model generation unit 23. Meanwhile, the uncertainties are great at points near the virtual data. The uncertainties are great because the variance $\sigma^2$ of the likelihood corresponding to the virtual data has been set to a value greater than 0 by the model generation unit 23.

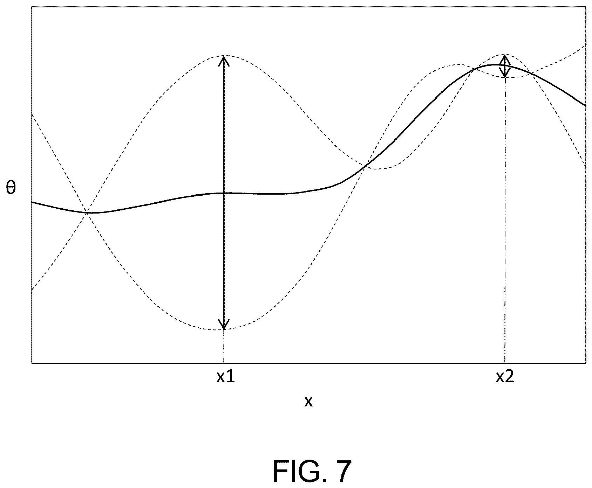

After the prediction model has been generated, as shown in Fig. 3, an experimental design is created (step S5). In creating the experimental design, first, the manufacturing condition calculation unit 25 calculates a third manufacturing condition for the sample on the basis of the prediction model generated by the model generation unit 23. Fig. 7 is a diagram showing an example of calculation of a third manufacturing condition based on a prediction model in the example of operation of the physical property prediction apparatus 1 according to the first embodiment. In the example shown in Fig. 7, the manufacturing condition calculation unit 25 calculates, as the third manufacturing condition, a manufacturing condition where the uncertainties of the physical property in the prediction model reach their maximums and a manufacturing condition where the uncertainties of the physical property in the prediction model reach their minimums. More specifically, the manufacturing condition calculation unit 25 calculates, as the third manufacturing condition, a value x1 of the parameter x at which the uncertainties of the physical property θ reach their maximums and a value x2 of the parameter x at which the uncertainties of the physical property θ reach their minimums. Note here that in a case where the uncertainties of the physical property are at their maximums, there is a possibility, albeit with a low probability, that a very satisfactory physical property may be attained. Further, in a case where the uncertainties of the physical property are at their minimums, there is high probability that a satisfactory physical property may be attained. Accordingly, by calculating, as the third manufacturing condition, the value x1 of the parameter x at which the uncertainties of the physical property θ reach their maximums and the value x2 of the parameter x at which the uncertainties of the physical property θ reach their minimums, the number of times that the sample is prepared until a desired physical property is attained can be reduced Next, in creating the experimental design, the user creates a specific plan for manufacturing a sample under the third manufacturing condition thus calculated.

After the experimental design has been created, as shown in Fig. 3, the user conducts an experiment (step S6). In conducting the experiment, first, the user manufactures a sample in accordance with the experimental design, i.e. under the third manufacturing condition. The sample is manufactured by using a manufacturing apparatus (not illustrated). After the sample has been manufactured, the user measures a physical property of the sample thus manufactured. The physical property of the sample is measured by using a measuring instrument (not illustrated) suited to the physical property.

In a case where, as a result of the experiment, the desired physical property has been attained (step S7: Yes), the process is ended. On the other hand, in a case where, as a result of the experiment, the desired physical property has not been attained (step S7: No) and an additional experiment is possible (step S8: Yes), the user inputs to the input screen the actual measurement data obtained by the experiment (step S3). Along with the inputting of the actual measurement data obtained by the experiment, the model generation unit 23 updates the prediction model (step S4).

As mentioned above, in the first embodiment, the actual measurement data acquisition unit 21 acquires actual measurement data including a first manufacturing condition for a sample and an actually measured value obtained by actually measuring a physical property of the sample manufactured under the first manufacturing condition. Further, the virtual data acquisition unit 22 acquires virtual data including a second manufacturing condition for the sample and a virtual value obtained by virtualizing a physical property of the sample that corresponds to the second manufacturing condition. Further, the model generation unit 23 generates a prediction model of the physical property on the basis of the actual measurement data acquired by the actual measurement data acquisition unit 21 and the virtual data acquired by the virtual data acquisition unit 22. Specifically, the model generation unit 23 generates such a prediction model that has a first uncertainty of the physical property with respect to the actual measurement data and a second uncertainty of the physical property with respect to the virtual data. The second uncertainty of the sample is greater than the first uncertainty of the sample.

This makes it possible to, even in a case where there are only few or no actual measurement data acquired, to appropriately create a sequential experimental design by making effective use of virtual data reflecting tacit knowledge such as experience and intuition in material development. Further, even in a case where the number of times that an experiment can be conducted is limited and it is difficult to perform sample preparation and a measurement the number of times required to construct an initial prediction model, a material having a desired physical property can be developed. Further, also in a case where an experiment can be conducted sufficiently many times, the number of times that sample preparation is conducted until a desired physical property is attained can be reduced by utilizing tacit knowledge that is not expressed by the actual measurement data. Accordingly, the first embodiment makes it possible to appropriately and efficiently develop a material having a desired physical property.

Further, in the first embodiment, the virtual data acquisition unit 22 may acquire, as the virtual value, a physical property value of the sample that is predicted in a case where a value of one or more of a plurality of parameters included in the second manufacturing condition is extremely small or large.

Accordingly, virtual data useful in the generation of the prediction model can be acquired with a simple technique.

Further, in the first embodiment, the virtual data acquisition unit 22 may acquire, as the virtual value, a physical property value of a substance that is similar in raw material or process to the sample.

Accordingly, virtual data useful in the generation of the prediction model can be acquired with a simple technique.

Further, in the first embodiment, the first uncertainty of the physical property is 0.

This makes it possible to generate a prediction model that makes it possible to easily search for an optimum manufacturing condition.

Further, in the first embodiment, the physical property prediction apparatus 1 further includes an input screen generation unit 24 that generates an input screen through which to input the actual measurement data and the virtual data. Further, the actual measurement data acquisition unit 21 acquires the actual measurement data inputted to the input screen generated by the input screen generation unit 24. Further, the virtual data acquisition unit 22 acquires the virtual data inputted to the input screen generated by the input screen generation unit 24.

This makes it possible to a prediction model reflecting the will of a user.

Further, in the first embodiment, the model generation unit 23 generates the prediction model using Bayesian inference according to mathematical formulas (1) and (2). That is, the model generation unit 23 generates such a prediction model that has a first variance with respect to the actual measurement data and has a second variance with respect to the virtual data. The second variance that is greater than the first variance.

This makes it possible to generate a prediction model that makes it possible to easily search for an optimum manufacturing condition.

Further, in the first embodiment, the manufacturing condition calculation unit 25 calculates a third manufacturing condition for the sample on the basis of the prediction model generated by the model generation unit 23.

This makes it possible to search for an optimum manufacturing condition on the basis of the prediction model.

Further, in the first embodiment, the third manufacturing condition includes at least one of a manufacturing condition where the uncertainties of the physical property in the prediction model reach their maximums and a manufacturing condition where the uncertainties of the physical property in the prediction model reach their minimums.

This makes it possible to reduce the number of times that the sample is prepared until a desired physical property is attained, making it possible to efficiently develop a material having a desired physical property.

Further, in the first embodiment, the actual measurement data acquisition unit 21 further acquires second actual measurement data including the third manufacturing condition calculated by the manufacturing condition calculation unit 25 and a second actually measured value obtained by actually measuring a physical property of the sample manufactured under the third manufacturing condition. Further, the model generation unit 23 updates the prediction model on the basis of the second actual measurement data acquired by the actual measurement data acquisition unit 21.

This makes it possible to optimize the experimental design, thus making it possible to appropriately develop a material having a desired physical property.

Further, in the first embodiment, the model generation unit 23 does not update the prediction model in a case where the second actually measured value is a set optimum value.

This makes it possible to reduce the number of times that the sample is prepared.

[Second Embodiment]

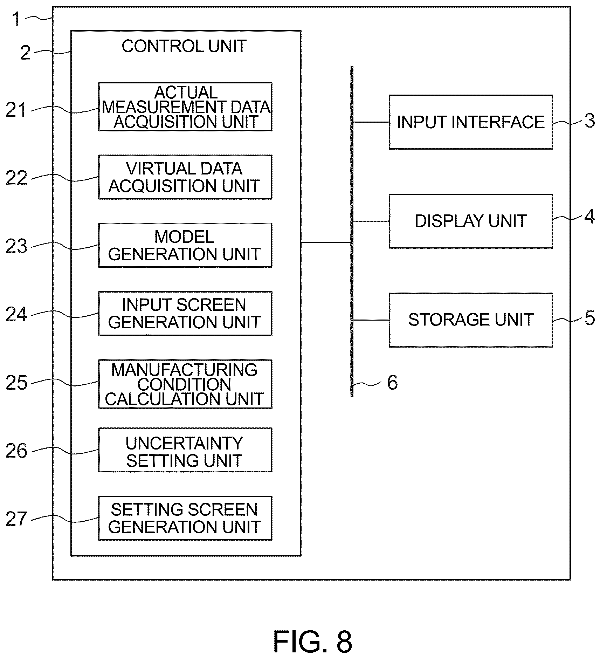

Next, a second embodiment in which a prediction model is generated on the basis of set uncertainties is described with a focus on differences from the first embodiment. Fig. 8 is a block diagram showing a physical property prediction apparatus 1 according to the second embodiment.

As shown in Fig. 8, the control unit 2 of the physical property prediction apparatus 1 according to the second embodiment further includes an uncertainty setting unit 26 and a setting screen generation unit 27 in addition to the components of the first embodiment.

The uncertainty setting unit 26 sets the first uncertainty of the physical property and the second uncertainty of the physical property. The model generation unit 23 generates the prediction model on the basis of the first uncertainty of the physical property and the second uncertainty of the physical property as set by the uncertainty setting unit 26.

The setting screen generation unit 27 generates a setting screen through which to set the first uncertainty of the physical property and the second uncertainty of the physical property. The setting screen generation unit 27 displays on the display unit 4 the setting screen thus generated. The uncertainty setting unit 26 sets the first uncertainty of the physical property and the second uncertainty of the physical property in accordance with an input operation performed on the setting screen generated by the setting screen generation unit 27. More specifically, the uncertainty setting unit 26 sets the first uncertainty of the physical property and the second uncertainty of the physical property in accordance with an input operation performed with the input interface 3 on the setting screen displayed on the display unit 4. The setting screen generation unit 27 may display the setting screen at the same time as the input screen generation unit 24 displays the input screen. Alternatively, the setting screen generation unit 27 may display the setting screen at a timing that is different from the timing at which the input screen generation unit 24 displays the input screen.

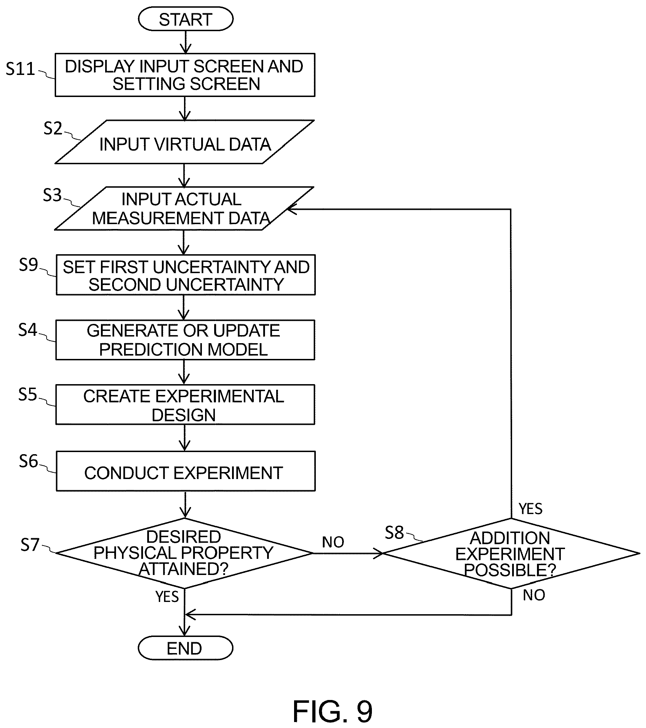

Fig. 9 is a flow chart showing an example of operation of the physical property prediction apparatus 1 according to the second embodiment. As shown in Fig. 9, in the second embodiment, the setting screen generation unit 27 displays the setting screen at the same time as the input screen generation unit 24 displays the input screen (step S11).

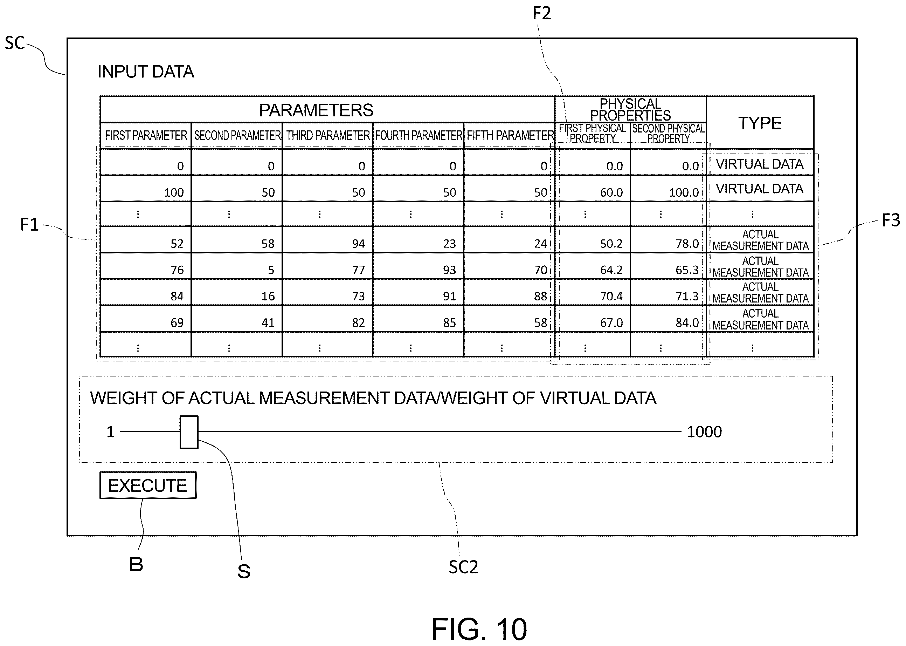

Fig. 10 is a diagram showing an example of an input screen SC in the example of operation of the physical property prediction apparatus according to the second embodiment. In the example shown in Fig. 10, the setting screen generation unit 27 displays a setting screen SC2 on the input screen. The setting screen SC2 has a slider bar S that enables the inputting of the weight of the actual measurement data and the weight of the virtual data. By inputting the ratio of the weight of the actual measurement data to the weight of the virtual data by sliding the slider bar S, the first uncertainty and the second uncertainty can be set in correspondence with the ratio thus inputted.

As shown in Fig. 9, in the second embodiment, the uncertainty setting unit 26 sets the first uncertainty and the second uncertainty in accordance with an input operation performed on the setting screen (step S9).

The model generation unit 23 generates a prediction model on the basis of the virtual data and the actual measurement data thus inputted and the first uncertainty and the second uncertainty thus set (step S4).

As mentioned above, in the second embodiment, the uncertainty setting unit 26 sets the first uncertainty of the physical property and the second uncertainty of the physical property. Further, the model generation unit 23 generates the prediction model on the basis of the first uncertainty of the physical property and the second uncertainty of the physical property as set by the uncertainty setting unit 26.

This makes it possible to calculate a prediction model reflecting the will of a user, thus making it possible to improve the degree of freedom of search for a manufacturing condition.

Further, in the second embodiment, the setting screen generation unit 27 generates a setting screen through which to set the first uncertainty of the physical property and the second uncertainty of the physical property. Further, the uncertainty setting unit 26 sets the first uncertainty of the physical property and the second uncertainty of the physical property in accordance with an input operation performed on the setting screen generated by the setting screen generation unit 27.

This makes it possible to easily set the first uncertainty and the second uncertainty through the setting screen.

[Third Embodiment]

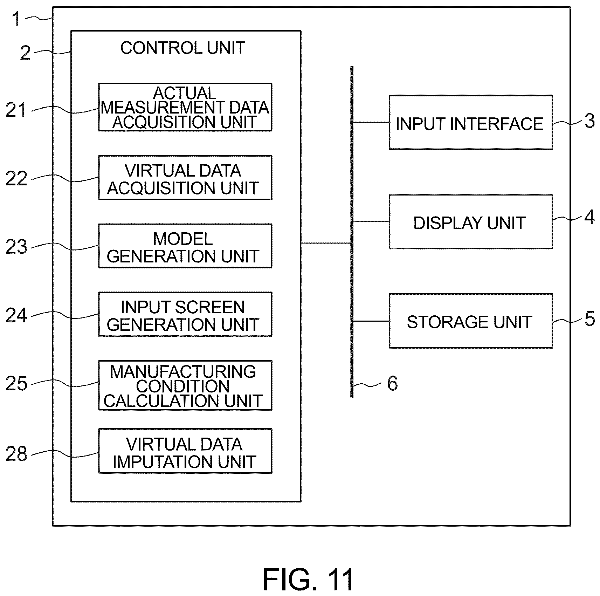

Next, a third embodiment in which the virtual data are imputed is described with a focus on differences from the first embodiment. Fig. 11 is a block diagram showing a physical property prediction apparatus 1 according to the third embodiment.

As shown in Fig. 11, the control unit 2 of the physical property prediction apparatus 1 according to the third embodiment further includes a virtual data imputation unit 28 in addition to the components of the first embodiment.

The virtual data imputation unit 28 imputes the virtual data. In a case where the virtual data inputted to the input screen are not complete data but partially missing data, the virtual data imputation unit 28 imputes the virtual data by complementing the missing data.

The virtual data acquisition unit 22 acquires the virtual data imputed by the virtual data imputation unit 28.

The virtual data imputation unit 28 may impute the virtual data when one or more parameters, included in the plurality of parameters included in the second manufacturing condition, that have extremely small or large values and the virtual value predicted on the basis of the one or more parameters are inputted to the input screen. Specifically, virtual data imputation unit 28 may impute the second manufacturing condition of the virtual data by calculating a value of one of the plurality of parameters other than the one or more parameters.

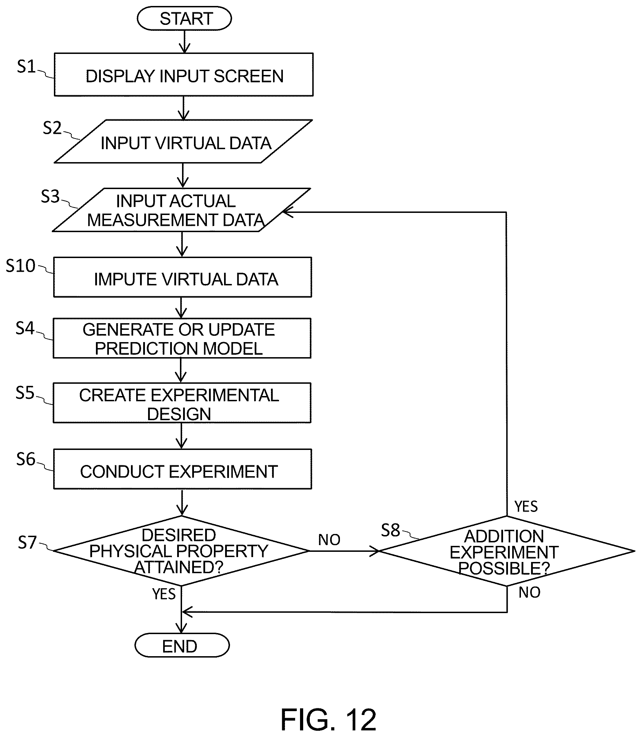

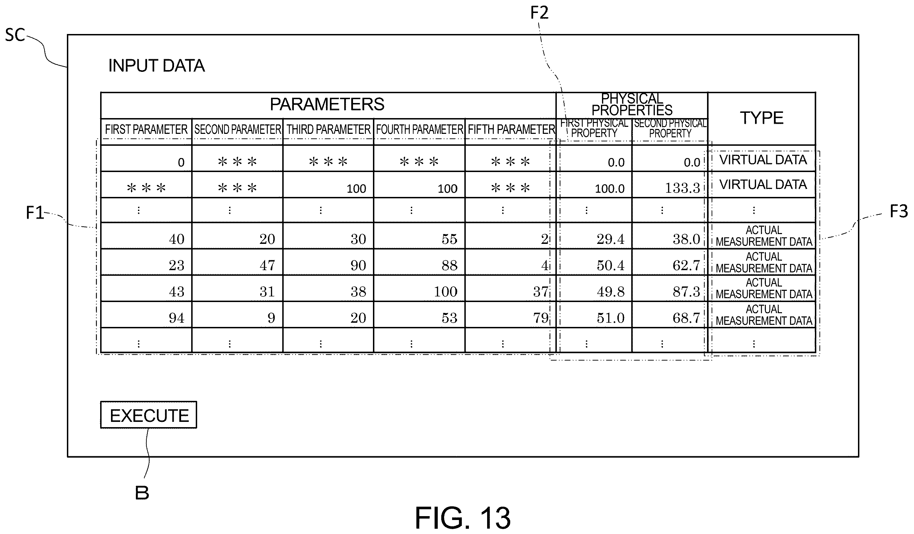

Fig. 12 is a flow chart showing an example of operation of the physical property prediction apparatus 1 according to the third embodiment. Fig. 13 is a diagram showing an example of an input screen in the example of operation of the physical property prediction apparatus 1 according to the third embodiment.

As shown in Fig. 12, in the third embodiment, the virtual data imputation unit 28 imputes the virtual data in a case where the virtual data inputted to the input screen are not complete data (step S10).

For example, when, of m parameters $x_1$ $x_2$, ..., and $x_m$ included in the second manufacturing condition, one parameter $x_1$ assumes an extremely small or large value $x_1^*$, it may be able to be predicted by the user's tacit knowledge that the physical property value of the sample is $\theta^*$. In a case where it can be predicted that the physical property value is $\theta^*$, the user inputs $x_1^*$ as the value of the parameter $x_1$ to the input screen, inputs $\theta^*$ as the physical property value to the input screen, and does not input the values of the parameters other than $x_1$. In the example shown in Fig. 13, 0, which is extremely small, is inputted as the value of the first parameter to the input screen SC. Further, 0.0 is inputted as the first physical property and the second physical property to the input screen SC. Further, in the input screen SC, the values of the parameters other than the first parameter are set as undefined, ***. i.e.

In a case where the values of the parameters other than $x_1$ are not inputted, the virtual data become incomplete. In a case where the virtual data become incomplete, the virtual data imputation unit 28 imputes the virtual data by calculating and adding the values of the parameters $x_2$, ..., and $x_m$ other than $x_1$. For example, the virtual data imputation unit 28 divides, at a k level, the respective search areas of the parameters $x_2$, ..., and $x_m$ determined in advance. Then, the virtual data imputation unit 28 calculates the values of the parameters $x_2$, ..., and $x_m$ in $k^{m-1}$ patterns by combining values selected separately from each of the divided areas divided at the k level. Alternatively, the virtual data imputation unit 28 may calculate the values of the parameters $x_2$, ..., and $x_m$ by generating m-1 dimensional uniform random numbers instead of the method of calculating involving the use of the k level.

Further, when, of the m parameters $x_1$ $x_2$, ..., and $x_m$ included in the second manufacturing condition, two or more and m-1 or less parameters assume extremely small or large values, the physical property value of the sample may be able to be predicted by the user's tacit knowledge. In a case where the physical property value can be predicted, the user inputs the values of the two or more and m-1 or less parameters to the input screen, inputs the predicted physical property value to the input screen, and does not input the values of the parameters other than the two or more and m-1 or less parameters. In the example shown in Fig. 13, 100, which is extremely large, is inputted as the value of a third parameter to the input screen SC. Further, 100, which is extremely large, is inputted as the value of a fourth parameter to the input screen SC. Further, 100.0 is inputted as the first physical property to the input screen SC. Further, 133.3 is inputted as the second physical property to the input screen SC. Further, in the input screen SC, the values of the parameters other than the third and fourth parameters are set as undefined.

In a case where the values of the parameters other than the two or more and m-1 or less parameters are not inputted, the virtual data become incomplete. In a case where the virtual data become incomplete, for example, the virtual data imputation unit 28 divides, at a k level, the respective search areas of the parameters other than the two or more and m-1 or less parameters. Then, the virtual data imputation unit 28 calculates the values of the parameters by combining values selected separately from each of the divided areas divided at the k level.

In a case where actual measurement data of a substance that is similar to the sample can serve as a useful reference, the user may input the actual measurement data of the substance as virtual data to the input screen.

In a case where complete virtual data has been inputted to the input screen, the virtual data imputation unit 28 does not impute the virtual data.

As described above, in the third embodiment, the virtual data imputation unit 28 imputes the virtual data. Further, the virtual data acquisition unit 22 acquires the virtual data imputed by the virtual data imputation unit 28.

This makes it possible to efficiently generate the virtual data.

Further, in the third embodiment, the virtual data imputation unit 28 imputes the second manufacturing condition of the virtual data by, when one or more parameters, included in the plurality of parameters included in the second manufacturing condition, that have extremely small or large values and the virtual value predicted on the basis of the one or more parameters are inputted to the input screen, calculating a value of one of the plurality of parameters other than the one or more parameters.

This makes it possible to efficiently impute the virtual data.

[Fourth Embodiment]

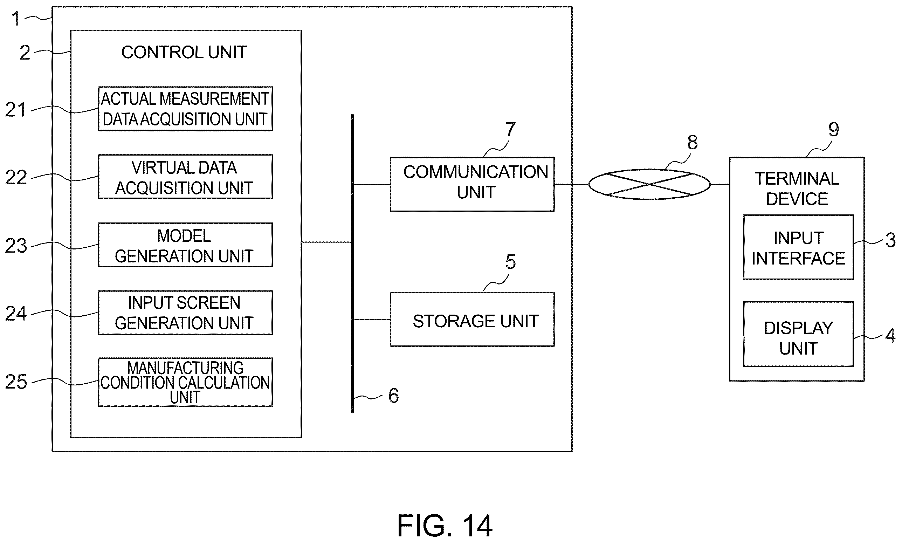

Next, a fourth embodiment in which the input screen is provided to a user's terminal device is described with a focus on differences from the first embodiment. Fig. 14 is a block diagram showing a physical property prediction apparatus 1 according to the fourth embodiment.

Fig. 1 has shown an example in which the input interface 3 and the display unit 4 are provided in the physical property prediction apparatus 1. In the example shown in Fig. 14 as opposed to the example shown in Fig. 1, the input interface 3 and the display unit 4 are provided in a user's terminal device 9. The physical property prediction apparatus 1 and the terminal device 9 are communicably connected to each other via a network 8.

The physical property prediction apparatus 1 includes a communication unit 7. The communication unit 7 is an interface for communicating with the terminal device 9 via the network 8. Possible examples of the communication unit 7 include a network card and a network adaptor. The communication unit 7 sends to the terminal device 9 the input screen generated by the input screen generation unit 24. The communication unit 7 receives from the terminal device 9 the actual measurement data and the virtual data inputted to the input screen.

The actual measurement data acquisition unit 21 acquires the actual measurement data received by the communication unit 7. The virtual data acquisition unit 22 acquires the virtual data received by the communication unit 27.

The model generation unit 23 may send via the communication unit 7 to the terminal device 9 the prediction model generated by the model generation unit 23. The prediction model sent to the terminal device 9 may be displayed on the display unit 4 of the terminal device 9.

The manufacturing condition calculation unit 25 may send via the communication unit 7 to the terminal device 9 the manufacturing condition calculated by the manufacturing condition calculation unit 25. The manufacturing condition sent to the terminal device 9 may be displayed on the display unit 4 of the terminal device 9.

The fourth embodiment allows a user who uses the terminal device 9 to search for an optimum manufacturing condition through the input screen provided from the physical property prediction apparatus 1, i.e. the server.

While several embodiments of the present disclosure have been described, each embodiment is presented as an example and is not intended to limit the scope of the disclosure. Each embodiment can be implemented in other various forms, and various omissions, substitutions, and alterations can be made without departing from the spirit of the present disclosure. Each embodiment and each modification of each embodiment are encompassed in the scope and spirit of the present disclosure and encompassed in the scope of the present disclosure and equivalents of the present disclosure as recited in the claims.

**Claims**

1. A physical property prediction apparatus comprising:

    an actual measurement data acquisition unit that acquires actual measurement data including a first manufacturing condition for a sample and an actually measured value obtained by actually measuring a physical property of the sample manufactured under the first manufacturing condition;
    a virtual data acquisition unit that acquires virtual data including a second manufacturing condition for the sample and a virtual value obtained by virtualizing a physical property of the sample that corresponds to the second manufacturing condition; and
    a model generation unit that, on the basis of the actual measurement data acquired by the actual measurement data acquisition unit and the virtual data acquired by the virtual data acquisition unit, generates such a prediction model of the physical property that has a first uncertainty of the physical property with respect to the actual measurement data and a second uncertainty of the physical property with respect to the virtual data, the second uncertainty of the sample being greater than the first uncertainty of the sample.

2. The physical property prediction apparatus according to claim 1, wherein the virtual data acquisition unit acquires, as the virtual value, a physical property value of the sample that is predicted in a case where a value of at least one of a plurality of parameters included in the second manufacturing condition is extremely small or large.

3. The physical property prediction apparatus according to claim 1, wherein the virtual data acquisition unit acquires, as the virtual value, a physical property value of a substance that is similar in raw material or process to the sample.

4. The physical property prediction apparatus according to claim 1, wherein the first uncertainty of the physical property is 0.

5. The physical property prediction apparatus according to claim 1, further comprising an input screen generation unit that generates an input screen through which to input the actual measurement data and the virtual data, wherein

    the actual measurement data acquisition unit acquires the actual measurement data inputted to the input screen generated by the input screen generation unit, and
    the virtual data acquisition unit acquires the virtual data inputted to the input screen generated by the input screen generation unit.

6. The physical property prediction apparatus according to claim 5, further comprising a communication unit that sends to a user's terminal device the input screen generated by the input screen generation unit and that receives from the terminal device the actual measurement data and the virtual data inputted to the input screen,
wherein

the actual measurement data acquisition unit acquires the actual measurement data received by the communication unit, and
the virtual data acquisition unit acquires the virtual data received by the communication unit.

7. The physical property prediction apparatus according to claim 5, further comprising a virtual data imputation unit that imputes the virtual data,
wherein the virtual data acquisition unit acquires the virtual data imputed by the virtual data imputation unit.

8. The physical property prediction apparatus according to claim 7, wherein the virtual data imputation unit imputes the second manufacturing condition of the virtual data by, when one or more parameters, included in the plurality of parameters included in the second manufacturing condition, that have extremely small or large values and the virtual value predicted on the basis of the one or more parameters are inputted to the input screen, calculating a value of one of the plurality of parameters other than the one or more parameters.

9. The physical property prediction apparatus according to claim 1, further comprising an uncertainty setting unit that sets the first uncertainty of the physical property and the second uncertainty of the physical property,
wherein the model generation unit generates the prediction model on the basis of the first uncertainty of the physical property and the second uncertainty of the physical property as set by the uncertainty setting unit.

10. The physical property prediction apparatus according to claim 9, further comprising a setting screen generation unit that generates a setting screen through which to set the first uncertainty of the physical property and the second uncertainty of the physical property,
wherein the uncertainty setting unit sets the first uncertainty of the physical property and the second uncertainty of the physical property in accordance with an input operation performed on the setting screen generated by the setting screen generation unit.

11. The physical property prediction apparatus according to claim 1, wherein

the model generation unit generates the prediction model using Bayesian inference,
the first uncertainty is a first variance, and
the second uncertainty is a second variance that is greater than the first variance.

12. The physical property prediction apparatus according to claim 11, wherein the model generation unit generates the prediction model according to the following formulas:

[Math. 1]

$$\theta_i = \sum_{j=1}^{d} \alpha_j \phi_j(x_i) + \varepsilon_i$$

[Math. 2]

$$p\left(\{\alpha_j\}_{j=1}^d \middle| \{x_i\}_{i=1}^n, \{\theta_i\}_{i=1}^n\right) = \frac{p\left(\{\theta_i\}_{i=1}^n \middle| \{x_i\}_{i=1}^n, \{\alpha_j\}_{j=1}^d\right) p\left(\{\alpha_j\}_{j=1}^d\right)}{\int p\left(\{\theta_i\}_{i=1}^n \middle| \{x_i\}_{i=1}^n, \{\alpha_j\}_{j=1}^d\right) p\left(\{\alpha_j\}_{j=1}^d\right) d\alpha_1 \cdots d\alpha_d}$$

$$= \frac{\left(\prod_{i=1}^n p\left(\theta_i \middle| x_i, \{\alpha_j\}_{j=1}^d\right)\right) \cdot p\left(\{\alpha_j\}_{j=1}^d\right)}{\int \left(\prod_{i=1}^n p\left(\theta_i \middle| x_i, \{\alpha_j\}_{j=1}^d\right)\right) \cdot p\left(\{\alpha_j\}_{j=1}^d\right) d\alpha_1 \cdots d\alpha_d}$$

where $\theta_\ell$ is a physical property in the ith datum of the actual measurement data and the virtual data, $x_i$ is a manufacturing condition in the ith datum of the actual measurement data and the virtual data, $\phi_j(\cdot)$ is a basis function, $\varepsilon_i$ is an error in the physical property in the ith datum of the actual measurement data and the virtual data, $\alpha_j$ is a parameter of the prediction model, $p\left(\{\alpha_j\}_{j=1}^d\right)$ is a prior distribution, $p\left(\{\alpha_j\}_{j=1}^d \middle| \{x_i\}_{i=1}^n, \{\theta_i\}_{i=1}^n\right)$ is a posterior distribution, $p\left(\theta_i \middle| x_i, \{\alpha_j\}_{j=1}^d\right)$ is a likelihood corresponding to the ith datum and is a value equivalent to $p\left(\theta_i \middle| x_i, \{\alpha_j\}_{j=1}^d\right) = N(\theta_i | \sum_{j=1}^d \alpha_j \phi_j(x_i), \sigma_i^2)$, $\sigma_i^2$ is an error variance of the physical property in the ith datum of the actual measurement data and the virtual data, and $\int p\left(\{y_i\}_{i=1}^n \middle| \{x_i\}_{i=1}^n, \{\alpha_j\}_{j=1}^d\right) p\left(\{\alpha_j\}_{j=1}^d\right) d\alpha_1 \cdots d\alpha_d$ is a marginal likelihood.

13. The physical property prediction apparatus according to any one of claims 1 to 12, further comprising a manufacturing condition calculation unit that calculates a third manufacturing condition for the sample on the basis of the prediction model generated by the model generation unit.

14. The physical property prediction apparatus according to claim 13, wherein the third manufacturing condition includes at least one of a manufacturing condition where the uncertainties of the physical property in the prediction model reach their maximums and a manufacturing condition where the uncertainties of the physical property in the prediction model reach their minimums.

15. The physical property prediction apparatus according to claim 13, wherein

the actual measurement data acquisition unit further acquires second actual measurement data including the third manufacturing condition calculated by the manufacturing condition calculation unit and a second actually measured value obtained by actually measuring a physical property of the sample manufactured under the third manufacturing condition, and
the model generation unit updates the prediction model on the basis of the second actual measurement data acquired by the actual measurement data acquisition unit.

16. The physical property prediction apparatus according to claim 15, wherein the model generation unit does not update the prediction model in a case where the second actually measured value is a set optimum value.

17. A physical property prediction method comprising the steps of:

acquiring actual measurement data including a first manufacturing condition for a sample and an actually measured value obtained by actually measuring a physical property of the sample manufactured under the first manufacturing condition;
acquiring virtual data including a second manufacturing condition for the sample and a virtual value obtained by virtualizing a physical property of the sample that corresponds to the second manufacturing condition; and
on the basis of the actual measurement data thus acquired and the virtual data thus acquired, generating such a prediction model of the physical property that has a first uncertainty of the physical property with respect to the actual measurement data and a second uncertainty of the physical property with respect to the virtual data, the second uncertainty of the sample being greater than the first uncertainty of the sample.

18. A physical property prediction program for causing a computer to execute procedures comprising the steps of:

acquiring actual measurement data including a first manufacturing condition for a sample and an actually measured value obtained by actually measuring a physical property of the sample manufactured under the first manufacturing condition;
acquiring virtual data including a second manufacturing condition for the sample and a virtual value obtained by virtualizing a physical property of the sample that corresponds to the second manufacturing condition; and
on the basis of the actual measurement data thus acquired and the virtual data thus acquired, generating such a prediction model of the physical property that has a first uncertainty of the physical property with respect to the actual measurement data and a second uncertainty of the physical property with respect to the virtual data, the second uncertainty of the sample being greater than the first uncertainty of the sample.

FIG. 1

1

2 — CONTROL UNIT

21 — ACTUAL MEASUREMENT
DATA ACQUISITION UNIT

22 — VIRTUAL DATA
ACQUISITION UNIT

23 — MODEL
GENERATION UNIT

24 — INPUT SCREEN
GENERATION UNIT

25 — MANUFACTURING
CONDITION CALCULATION
UNIT

INPUT INTERFACE — 3

DISPLAY UNIT — 4

STORAGE UNIT — 5

6

FIG. 2

FIG. 3

SC

INPUT DATA

| PARAMETERS | | | | | PHYSICAL PROPERTIES | | TYPE |
|---|---|---|---|---|---|---|---|
| FIRST PARAMETER | SECOND PARAMETER | THIRD PARAMETER | FOURTH PARAMETER | FIFTH PARAMETER | FIRST PHYSICAL PROPERTY | SECOND PHYSICAL PROPERTY | |
| 0 | 0 | 0 | 0 | 0 | 0.0 | 0.0 | VIRTUAL DATA |
| 100 | 50 | 50 | 50 | 50 | 60.0 | 100.0 | VIRTUAL DATA |
| ⋮ | ⋮ | ⋮ | ⋮ | ⋮ | ⋮ | ⋮ | ⋮ |
| 52 | 58 | 94 | 23 | 24 | 50.2 | 78.0 | ACTUAL MEASUREMENT DATA |
| 76 | 5 | 77 | 93 | 70 | 64.2 | 65.3 | ACTUAL MEASUREMENT DATA |
| 84 | 16 | 73 | 91 | 88 | 70.4 | 71.3 | ACTUAL MEASUREMENT DATA |
| 69 | 41 | 82 | 85 | 58 | 67.0 | 84.0 | ACTUAL MEASUREMENT DATA |
| ⋮ | ⋮ | ⋮ | ⋮ | ⋮ | ⋮ | ⋮ | ⋮ |

F2

F1

F3

EXECUTE

B

EP 4 726 725 A1

FIG. 4

FIG. 5

FIG. 6

FIG. 7

1

2 CONTROL UNIT

21 ACTUAL MEASUREMENT DATA ACQUISITION UNIT

INPUT INTERFACE — 3

22 VIRTUAL DATA ACQUISITION UNIT

23 MODEL GENERATION UNIT

DISPLAY UNIT — 4

24 INPUT SCREEN GENERATION UNIT

STORAGE UNIT — 5

25 MANUFACTURING CONDITION CALCULATION UNIT

~ 6

26 UNCERTAINTY SETTING UNIT

27 SETTING SCREEN GENERATION UNIT

FIG. 8

START

S11 — DISPLAY INPUT SCREEN AND
SETTING SCREEN

S2 — INPUT VIRTUAL DATA

S3 — INPUT ACTUAL
MEASUREMENT DATA

S9 — SET FIRST UNCERTAINTY AND
SECOND UNCERTAINTY

S4 — GENERATE OR UPDATE
PREDICTION MODEL

S5 — CREATE EXPERIMENTAL
DESIGN

S6 — CONDUCT EXPERIMENT

S7 — DESIRED
PHYSICAL PROPERTY
ATTAINED?

NO

S8 — ADDITION
EXPERIMENT
POSSIBLE?

YES

NO

YES

END

FIG. 9

INPUT DATA

| | PARAMETERS | | | | PHYSICAL PROPERTIES | | TYPE |
|---|---|---|---|---|---|---|---|
| FIRST PARAMETER | SECOND PARAMETER | THIRD PARAMETER | FOURTH PARAMETER | FIFTH PARAMETER | FIRST PHYSICAL PROPERTY | SECOND PHYSICAL PROPERTY | |
| 0 | 0 | 0 | 0 | 0 | 0.0 | 0.0 | VIRTUAL DATA |
| 100 | 50 | 50 | 50 | 50 | 60.0 | 100.0 | VIRTUAL DATA |
| ⋮ | ⋮ | ⋮ | ⋮ | ⋮ | ⋮ | ⋮ | ⋮ |
| 52 | 58 | 94 | 23 | 24 | 50.2 | 78.0 | ACTUAL MEASUREMENT DATA |
| 76 | 5 | 77 | 93 | 70 | 64.2 | 65.3 | ACTUAL MEASUREMENT DATA |
| 84 | 16 | 73 | 91 | 88 | 70.4 | 71.3 | ACTUAL MEASUREMENT DATA |
| 69 | 41 | 82 | 85 | 58 | 67.0 | 84.0 | ACTUAL MEASUREMENT DATA |
| ⋮ | ⋮ | ⋮ | ⋮ | ⋮ | ⋮ | ⋮ | ⋮ |

WEIGHT OF ACTUAL MEASUREMENT DATA/WEIGHT OF VIRTUAL DATA

1 ——————————————————————— 1000

EXECUTE

FIG. 10

1

2 — CONTROL UNIT

21 — ACTUAL MEASUREMENT DATA ACQUISITION UNIT

22 — VIRTUAL DATA ACQUISITION UNIT

23 — MODEL GENERATION UNIT

24 — INPUT SCREEN GENERATION UNIT

25 — MANUFACTURING CONDITION CALCULATION UNIT

28 — VIRTUAL DATA IMPUTATION UNIT

INPUT INTERFACE — 3

DISPLAY UNIT — 4

STORAGE UNIT — 5

6

FIG. 11

START

S1 — DISPLAY INPUT SCREEN

S2 — INPUT VIRTUAL DATA

S3 — INPUT ACTUAL MEASUREMENT DATA

S10 — IMPUTE VIRTUAL DATA

S4 — GENERATE OR UPDATE PREDICTION MODEL

S5 — CREATE EXPERIMENTAL DESIGN

S6 — CONDUCT EXPERIMENT

S7 — DESIRED PHYSICAL PROPERTY ATTAINED?

NO

S8 — ADDITION EXPERIMENT POSSIBLE?

YES

NO

YES

END

FIG. 12

SC — INPUT DATA

| PARAMETERS | | | | | PHYSICAL PROPERTIES | | TYPE |
|---|---|---|---|---|---|---|---|
| FIRST PARAMETER | SECOND PARAMETER | THIRD PARAMETER | FOURTH PARAMETER | FIFTH PARAMETER | FIRST PHYSICAL PROPERTY | SECOND PHYSICAL PROPERTY | |
| 0 | * * * | * * * | * * * | * * * | 0.0 | 0.0 | VIRTUAL DATA |
| * * * | * * * | 100 | 100 | * * * | 100.0 | 133.3 | VIRTUAL DATA |
| ⋮ | ⋮ | ⋮ | ⋮ | ⋮ | ⋮ | ⋮ | ⋮ |
| 40 | 20 | 30 | 55 | 2 | 29.4 | 38.0 | ACTUAL MEASUREMENT DATA |
| 23 | 47 | 90 | 88 | 4 | 50.4 | 62.7 | ACTUAL MEASUREMENT DATA |
| 43 | 31 | 38 | 100 | 37 | 49.8 | 87.3 | ACTUAL MEASUREMENT DATA |
| 94 | 9 | 20 | 53 | 79 | 51.0 | 68.7 | ACTUAL MEASUREMENT DATA |
| ⋮ | ⋮ | ⋮ | ⋮ | ⋮ | ⋮ | ⋮ | ⋮ |

F2 F1 F3

EXECUTE

B

FIG. 13

FIG. 14

## INTERNATIONAL SEARCH REPORT

| International application No. |
| --- |
| **PCT/JP2024/019633** |

| | |
| --- | --- |
| **A.** | **CLASSIFICATION OF SUBJECT MATTER** |
| | ***G16C 60/00***(2019.01)i; ***G16C 20/70***(2019.01)i |
| | FI: G16C60/00; G16C20/70 |

According to International Patent Classification (IPC) or to both national classification and IPC

| | |
| --- | --- |
| **B.** | **FIELDS SEARCHED** |

Minimum documentation searched (classification system followed by classification symbols)

G06Q10/00-99/00; G16C10/00-99/00; G16Z99/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

| | |
| --- | --- |
| **C.** | **DOCUMENTS CONSIDERED TO BE RELEVANT** |

| Category* | Citation of document, with indication, where appropriate, of the relevant passages | Relevant to claim No. |
| --- | --- | --- |
| A | JP 2022-65466 A (NIKON CORPORATION) 27 April 2022 (2022-04-27) paragraphs [0009]-[0014], [0020]-[0029], [0043], [0066]-[0068], [0092] | 1-18 |
| A | JP 2022-14618 A (HITACHI, LTD.) 20 January 2022 (2022-01-20) paragraphs [0019], [0033]-[0034], [0037], [0047] | 1-18 |
| A | JP 2022-82064 A (FUJITSU LIMITED) 01 June 2022 (2022-06-01) paragraphs [0012]-[0014], [0017], [0022]-[0025], [0056], [0069] | 1-18 |
| A | US 7729965 B1 (FANNIE, Mae) 01 June 2010 (2010-06-01) column 2, line 37 to column 4, line 3, column 4, lines 19-57, column 5, lines 33-41 | 1-18 |

☐ Further documents are listed in the continuation of Box C.　　☑ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **05 August 2024** | **13 August 2024** |

| Name and mailing address of the ISA/JP | Authorized officer |
| --- | --- |
| **Japan Patent Office (ISA/JP) 3-4-3 Kasumigaseki, Chiyoda-ku, Tokyo 100-8915 Japan** | |
| | Telephone No. |

Form PCT/ISA/210 (second sheet) (July 2022)

**INTERNATIONAL SEARCH REPORT**
Information on patent family members

International application No.

**PCT/JP2024/019633**

| Patent document cited in search report | | | Publication date (day/month/year) | Patent family member(s) | Publication date (day/month/year) |
|---|---|---|---|---|---|
| JP | 2022-65466 | A | 27 April 2022 | (Family: none) | |
| JP | 2022-14618 | A | 20 January 2022 | (Family: none) | |
| JP | 2022-82064 | A | 01 June 2022 | US 2022/0164685 A1 paragraphs [0034]-[0037], [0043], [0053]-[0057], [0128], [0153] EP 4002376 A1 CN 114520029 A | |
| US | 7729965 | B1 | 01 June 2010 | (Family: none) | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2021200281 A1

- WO 2020188971 A1